# EUROPÄISCHE PATENTANMELDUNG

(11) **EP 2 572 681 A1**
(43) Veröffentlichungstag der Anmeldung: **27.03.2013**
(21) Anmeldenummer: 12180765.5
(22) Anmeldetag: 17.08.2012
(51) Int. Cl.: A61F 2/915

(54) **Implantat und Verfahren zur Herstellung desselben**

(30) Priorität: 23.09.2011 US 201161538152 P
(71) Anmelder: Biotronik AG, 8180 Bülach (CH)
(72) Erfinder: Poehlmann, Stefanie, 18057 Rostock (DE); Amido, Alexandre, 18057 Rostock (DE)
(74) Vertreter: Lindner-Vogt, Karin L.

(57) **Zusammenfassung**

Die vorliegende Erfindung betrifft ein Implantat mit einer durchbrochenen, vorzugsweise hohlzylinderförmigen Grundstruktur, welche mindestens einen Abschnitt aufweist, wobei in jedem Abschnitt mindestens ein Steg angeordnet ist. Um eine homogenere Abdeckung der Wand des behandelten Gefäßes zu erzielen, einer guten Verzahnung mit der Wand des behandelten Körperhohlraums sowie eine bessere Verteilung der mechanischen Spannungen und Dehnungen zu erreichen, weist der Steg eine Vielzahl von gleichen, einfachen und miteinander verbundenen Elementen auf, deren Grundform zu der Form von Grundlinienelementen einer gedachten Grundlinie erster Ordnung, die ebenfalls in dem Abschnitt verläuft, im Wesentlichen ähnlich ist, wobei die gedachte Grundlinie erster Ordnung eine Bezugslinie für die Anordnung der Elemente darstellt und die Elemente höchstens halb so groß sind wie die Grundlinienelemente und stellt somit eine fraktale Struktur da.

## Beschreibung

Die vorliegende Erfindung betrifft ein Implantat, insbesondere eine intraluminale Endoprothese, mit einer durchbrochenen, vorzugsweise hohlzylinderförmigen Grundstruktur, welche mindestens einen Abschnitt aufweist, wobei in jedem Abschnitt mindestens ein Steg angeordnet ist.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents (Gefäß-Stents (vaskulärer Stent, einschließlich des Herzens und Herzklappenstents, z.B. Mitral-Stent, Pulmonalklappenstent), Gallengang-Stents), Endoprothesen zum Verschließen von persitierenden Foramen ovale (PFO), Stent Grafts zur Behandlung von Aneurysmen, Endoprothesen zum Verschließen eines ASD (Vorhofscheidewanddefekt, atrial septal defect) sowie Prothesen im Bereich des Hart- und Weichgewebes zu verstehen.

Heutzutage werden als Implantate besonders häufig Stents eingesetzt, die zur Behandlung von Stenosen (Gefäßverengungen) dienen. Sie weisen eine durchbrochene rohrförmige oder hohlzylinderförmige Grundstruktur auf, die an beiden Längsenden offen ist. Die Grundstruktur von herkömmlichen Stents setzt sich dabei häufig aus einzelnen Maschen zusammen, welche aus verschieden geformten Stegen (Struts), beispielsweise zick-zack- oder mäander-förmigen Stegen, gebildet werden. Ein derartiges Implantat wird häufig mittels eines Katheters in das zu behandelnde Gefäß eingesetzt und dient dazu, das Gefäß über einen längeren Zeitraum (Monate bis Jahre) zu stützen. Durch den Einsatz von Stents können verengte Bereiche in den Gefäßen erweitert werden, so dass ein Lumengewinn resultiert.

Für die Freigabe von Medikamenten aus derartigen Implantaten (zum Beispiel bei einem so genannten Drug Eluting Stent (DES)) in die Wand eines behandelten Gefäßes wird angestrebt, durch das Implantat eine große und möglichst homogene Abdeckung der Gefäßwand zu erreichen. Ferner soll ein Wandern eines Stents in vivo (z.B. in der Arterie) verhindert und die Festigkeit von Stents unter der Belastung des entsprechenden Gefäßes erhöht werden. Außerdem könnte die Röntgensichtbarkeit der Implantate verbessert werden, um die Platzierung der Implantate und ihr Verhalten in vivo von Zeit zu Zeit einfach überwachen zu können; die derzeit erhältlichen dünnen Streben von Stents verfügen häufig über eine nur geringe Röntgensichtbarkeit.

Die Aufgabe der vorliegenden Erfindung besteht somit darin, ein Implantat zu schaffen, das die oben angegebenen Verbesserungen aufweist.

Die obige Aufgabe wird durch ein Implantat mit den Merkmalen des Anspruchs 1 gelöst.

Der mindestens eine Steg eines Abschnitts eines erfindungsgemäßen Implantats weist insbesondere eine Vielzahl von gleichen, einfachen und miteinander verbundenen Elementen auf, deren Grundform zu der Form von Grundlinienelementen mindestens einer gedachten Grundlinie erster Ordnung, die ebenfalls in dem Abschnitt verläuft, im Wesentlichen ähnlich ist, wobei die mindestens eine gedachte Grundlinie erster Ordnung eine Bezugslinie für die Anordnung der Elemente darstellt und die Elemente höchstens halb so groß sind wie die Grundlinienelemente. Das zuletzt angegebene Merkmal bedeutet, dass die Elemente in mindestens einer Dimension, vorzugsweise in der Dimension entlang des Grundlinienelements, eine höchstens halb so große Abmessung aufweisen wie die Grundlinienelemente. Dies bedeutet, dass in dem Fall dass die Dimension des Elements entlang des Grundlinienelements als Länge des Elements bezeichnet wird, die Elemente höchstens die halbe Länge der Grundlinienelemente aufweisen. Die Höhe der Elemente kann variieren und beträgt bevorzugt ebenfalls die Hälfte der Höhe der Grundlinienelemente.

Mit anderen Worten wird der mindestens eine Steg des Implantats, welcher in einem Abschnitt angeordnet ist, aus mehreren Elementen gebildet. Diese Elemente weisen die gleiche Grundform auf, die der Form der sogenannten Grundlinienelemente im Wesentlichen ähnlich ist, aus welchen eine gedachte Grundlinie zusammengesetzt ist. Das Grundlinienelement dient in dem jeweiligen Abschnitt dazu, für die Elemente des mindestens einen Stegs eine Bezugslinie für die Anordnung zu bilden. Da die Grundform der Stegelemente und die Form der entlang der Grundlinie angeordneten Grundlinienelemente einander im Wesentlichen ähnlich ist, wird durch den erfindungsgemäßen Aufbau des Implantats gewissermaßen ein fraktaler (selbstähnlicher) Aufbau erzeugt. Die (kleinen) Elemente bilden einen Steg aus, der in seiner Struktur feingliedrig erscheint.

Mit der Bezugnahme auf den Begriff "Grundform" im Zusammenhang mit den Elementen der Stege soll neben der Verwendung von einfachen Formen auch verdeutlicht werden, dass die Stegbreite oder Stegdicke für die Betrachtung der Form des Stegelements und für den Vergleich mit der Form des Grundlinienelements außer Acht gelassen wird. Es wird demnach als Grundform beispielsweise die Form einer Mittellinie durch das Element oder einer oberen oder äußeren Begrenzungslinie oder einer unteren oder inneren Begrenzungslinie entlang des Elements betrachtet. Anders ausgedrückt wird bei einem z.B. rhombusförmigen Element eines Stegs lediglich die (mathematisch zweidimensionale) Form des Rhombus in Bezug auf das entsprechende Grundlinienelement betrachtet und die Breite und Dicke der Seiten des Rhombus vernachlässigt. Bevorzugte Grundformen sind der Wellenbogen, der vorzugsweise als Sinus-Bogen ausgebildet ist, der Rhombus und das Zick-Zack-Element.

Hierbei wird unter "im Wesentlichen ähnlich" nicht nur die strenge mathematischgeometrische Ähnlichkeit verstanden, sondern es werden auch die Fälle einbezogen, in denen die Form der Stegelemente und die Form der Grundlinienelemente die gleiche Grundform (Welle, Dreieck, Rhombus, Sinus etc.) aufweisen, jedoch in einzelnen Abmessungen, Krümmungsradien bzw. Winkeln verglichen mit der strengen mathematisch-geometrischen Ähnlichkeit voneinander abweichen. Beispielsweise können sowohl die Grundlinienelemente als auch die Stegelemente als Rhomben ausgebildet sein, wobei die einander entsprechenden Winkel der Grundlinienelement-Rhomben und die Stegelement-Rhomben nicht wie bei der strengen mathematisch-geometrischen Ähnlichkeit identisch, sondern etwas unterschiedlich gestaltet sind.

Bevorzugte Krümmungsradien für ein wellenbogenförmiges Element eines Stegs betragen zwischen 0,035 mm und 1 mm. Die Länge eines Elements eines Stegs (entlang des jeweiligen Grundelements) beträgt vorzugsweise zwischen 0,1 mm und 5 mm. Der Winkel zwischen zwei Seiten eines Rhombus-Elements oder eines Zick-Zack-Elements kann im Bereich zwischen 0,5° und 175° liegen.

Die erfindungsgemäße Gestaltung des Implantats hat entscheidenden Einfluss auf seine mechanischen Eigenschaften. Zunächst kann die Gestaltung in vielfacher Weise variiert werden und so verschiedene vorteilhafte Eigenschaften des Implantats kombiniert werden. Durch die Zusammensetzung der Stege aus den einzelnen Elementen kann eine höhere, homogenere Abdeckung der Wand des behandelten Körperhohlraums erzielt werden. Dies ist insbesondere für die Anwendung als Implantat mit einer Abgabe von Medikamenten von Vorteil. Weiter kann eine gute "Verzahnung" mit der Wand des behandelten Körperhohlraums erreicht werden, so dass eine Migration des Implantats in dem Körperhohlraum unterbunden wird. Aufgrund der kleingliedrigen Struktur des mindestens einen Stegs erlaubt dieser eine Bewegung in verschiedene Richtungen, so dass eine höhere Flexibilität des Implantats erzielt werden kann. Die mechanischen Spannungen und Dehnungen, die auf das Implantat im behandelten Körperhohlraum wirken, verteilen sich besser als bei konventionellen Design. Die Hebelverhältnisse werden verändert, was zu einer Erhöhung Festigkeit gegenüber dem konventionellen Implantat-Aufbau führt. Zudem wird eine Dilatationsreserve bereitgestellt, so dass eine zusätzliche Überdilatation ermöglicht wird. Ein Kostenvorteil insbesondere für geflochtene Stents (Braided Stents) ergibt sich daraus, dass der mindestens eine Steg aus jeweils den gleichen Elementen gebildet wird.

In einem Ausführungsbeispiel ist der mindestens eine Abschnitt des Implantats ringförmig oder helixförmig. Bei der Verwendung von ringförmigen Abschnitten ist es meist notwendig, die Stege der benachbarten Abschnitte miteinander mittels Verbindungsstegen zu verbinden. Bei einer helixförmigen Gestaltung des Implantatabschnitts kann es notwendig sein, die Stege der übereinanderliegenden Bereiche der Helix miteinander mittels Verbindungsstegen zu verbinden.

Entsprechend ist in einem weiteren Ausführungsbeispiel der mindestens eine Steg eines Abschnitts oder eines Bereichs des Abschnitts mit dem mindestens einen Steg des benachbarten Abschnitts oder eines benachbarten Bereichs des Abschnitts mittels mindestens eines Verbindungsstegs, der vorzugsweise S-förmig ausgebildet ist, verbunden. Hierdurch werden eine bessere Verteilung der Kräfte und eine höhere Stabilität erreicht. Vorzugsweise werden die Verbindungsstege dort mit dem jeweiligen Steg verbunden, wo die Krümmung des jeweiligen Stegs ein lokales Extremum (Minimum oder Maximum) aufweist. Alternativ kann ein Verbindungssteg im Bereich der Mitte (hinsichtlich der Länge des Stegs) zwischen zwei benachbarten Verbindungsbereichen zu benachbarten Verbindungsstegen entlang des Stegs angeordnet sein. Derartige Verbindungsstege werden als Mitte-zu-Mitte-Verbinder bezeichnet.

Besonders geeignete einfache und leicht herstellbare Elemente oder Grundlinienelemente für ein erfindungsgemäßes Implantat weisen die Form eines Wellenform-Bogens, vorzugsweise eines Sinus-Bogens, eines Rhombus, eines Dreiecks, eines Zick-Zack-Elements oder dergleichen auf.

Die gedachte Grundlinie erster Ordnung bildet eine Bezugslinie für die Anordnung für die Elemente des mindestens einen Stegs derart, dass die Elemente in dem Abschnitt entlang der mindestens einen gedachten Grundlinie erster Ordnung angeordnet sind.

Alternativ sind die Elemente entlang mindestens einer gedachten Grundlinie zweiter Ordnung angeordnet, wobei die gedachte Grundlinie zweiter Ordnung derart vorgesehen ist, dass sie die Grundlinienelemente aufweist, welche mit einer Größe von höchstens 50% entlang der mindestens einen Grundlinie erster Ordnung angeordnet sind. In diesem Fall ist es von Vorteil, wenn die Elemente des mindestens einen Stegs nur höchstens 25% der Größe der Grundlinienelemente haben, welche die mindestens eine gedachte Grundlinie erster Ordnung bilden (zumindest für die Dimension entlang der Grundlinie, die Dimension senkrecht zur Grundlinie kann variieren).

Als weitere Alternative sind die Elemente des mindestens einen Stegs entlang mindestens einer gedachten Grundlinie dritter Ordnung angeordnet, wobei zunächst mindestens eine Grundlinie zweiter Ordnung vorgesehen ist, welche die Grundlinienelemente aufweist, die mit einer Größe von höchstens 50% entlang der Grundlinie erster Ordnung angeordnet sind, und weiter mindestens eine Grundlinie dritter Ordnung vorgesehen ist, welche Grundlinienelemente aufweist, die mit einer Größe von höchstens 25% entlang der Grundlinie zweiter Ordnung angeordnet sind. In diesem Fall haben die Elemente des mindestens einen Stegs höchstens 12,5% der Größe der Grundlinienelemente der Grundlinie erster Ordnung (alle Abmessungen zumindest für die Dimension entlang der Grundlinie, die Dimension senkrecht zur Grundlinie kann variieren). Insbesondere die zuletzt genannte Variante der Anordnung der Elemente ergibt eine besonders feingliedrige Struktur des Implantats, durch die eine sehr homogene Abdeckung der Wand des jeweils behandelten Körperhohlraums erreicht wird.

Die Reihe der oben beschriebenen Ausführungsbeispiele von Implantaten mit mindestens einer gedachten Grundlinie erster Ordnung, mindestens einer gedachten Grundlinie zweiter Ordnung, mindestens einer gedachten Grundlinie dritter Ordnung kann entsprechend mit mindestens einer gedachten Grundlinie vierter Ordnung usw. analog fortgesetzt werden. Die Struktur der Implantatstege wird mit jeder gedachten Grundlinie höherer Ordnung immer feingliedriger.

Eine weitere Variabilität in der Gestaltung des Implantats kann dadurch erreicht werden, dass die Elemente oder Grundlinienelemente derart entlang der mindestens einen Grundlinie erster Ordnung oder der mindestens einen Grundlinie zweiter Ordnung oder der mindestens einen Grundlinie dritter Ordnung usw. angeordnet werden, dass die jeweiligen Grundlinie eine Zentrallinie oder eine obere oder linke Begrenzungslinie oder eine untere oder rechte Begrenzungslinie darstellt.

Das erfindungsgemäße Implantat wird nachfolgend in Ausführungsbeispielen anhand von Figuren erläutert. Dabei bilden alle beschriebenen und/oder bildlich dargestellten Merkmale den Gegenstand der Erfindung, auch unabhängig von ihrer Zusammenfassung in den Ansprüchen oder deren Rückbezüge.

Es zeigen schematisch:
- Fig. 1: drei Abschnitte eines ersten Ausführungsbeispiels eines erfindungsgemäßen Implantats in einer perspektivischen Ansicht von der Seite,
- Fig. 2: einen Ausschnitt einer Abwicklung eines zweiten Ausführungsbeispiels eines erfindungsgemäßen Implantats in einer Ansicht von der Seite,
- Fig. 3: einen Ausschnitt der gedachten Grundlinien erster Ordnung der Abwicklung (Fig. 3a) und einen Bereich einer Strebe (Fig. 3b) des in Fig. 2 dargestellten zweiten Ausführungsbeispiels in einer Ansicht von oben,
- Fig. 4: einen Ausschnitt der gedachten Grundlinien erster Ordnung (Fig. 4a) und einen Bereich einer Strebe (Fig.4b) einer Abwicklung eines dritten Ausführungsbeispiels eines erfindungsgemäßen Implantats in einer Ansicht von oben,
- Fig. 5: einen Ausschnitt einer Abwicklung eines vierten Ausführungsbeispiels eines erfindungsgemäßen Implantats in einer Ansicht von oben,
- Fig. 6: einen Ausschnitt einer Abwicklung eines fünften Ausführungsbeispiels eines erfindungsgemäßen Implantats in einer Ansicht von oben,
- Fig. 7: einen vergrößerten Ausschnitt der in Fig. 6 dargestellten Abwicklung,
- Fig. 8: einen Bereich einer Strebe (Fig. 8a) und einen Ausschnitt der Abwicklung (Fig. 8b) eines sechsten Ausführungsbeispiels eines erfindungsgemäßen Implantats in einer Ansicht von oben und
- Fig. 9: einen Bereich einer Strebe (Fig. 9a) und einen Ausschnitt der Abwicklung (Fig. 9b) eines siebten Ausführungsbeispiels eines erfindungsgemäßen Implantats in einer Ansicht von oben.

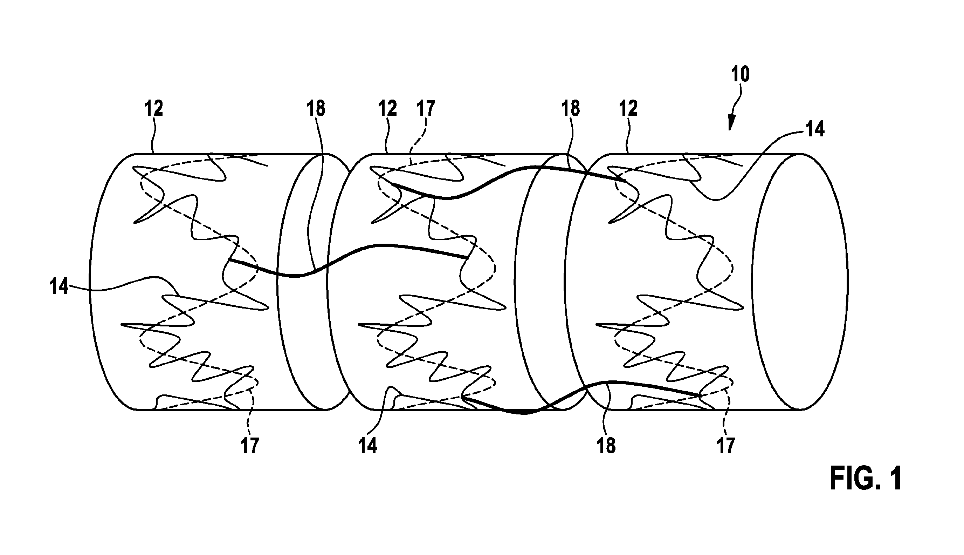

Fig. 1 zeigt drei Abschnitte 12 eines ersten Ausführungsbeispiels eines erfindungsgemäßen Implantats, das als Stent 10 ausgebildet ist. Jeder ringförmige Abschnitt 12 weist jeweils einen Steg 14 auf, der entlang einer gedachten, wellenförmigen Grundlinie 17 (gestrichelte Linie), wellenförmig mäandernd verläuft. Vorzugsweise ist die Grundlinie 17 sinusförmig gestaltet. Sowohl die Grundlinie 17 als auch der Steg 14 verläuft wellenförmig derart, dass sie Berge und Täler abwechselnd bzw. jeweils Krümmungsradien aufweisen, die abwechseln proximal und distal orientiert sind.

Es soll ausdrücklich darauf hingewiesen werden, dass die in Fig. 1 mit dem Bezugszeichen 12 versehenen ringförmigen Abschnitte lediglich gedachte Abschnitte sind, die zur Veranschaulichung der Stege 14 dienen.

Der Steg 14 ist aus wellenbogenförmigen Elementen zusammengesetzt, welche derart entlang der gedachten Grundlinie 17 angeordnet sind, dass die Grundlinie 17 eine gedachte Mittellinie bildet. Die Grundlinie 17 ist ebenfalls aus einzelnen Wellen-Bögen, vorzugsweise Sinus-Bögen, als Grundlinienelemente zusammengesetzt. Jeder Wellen-Bogen des Stegs 14 ist mit dem nächsten Wellen-Bogen des Stegs 14 verbunden. Auch die wellenbogen-förmigen Grundlinienelemente der Grundlinie 17 sind miteinander verbunden.

Die Länge eines wellenbogen- oder S-förmigen Stegelements beträgt ungefähr ein Sechstel der Länge eines wellenbogen- oder S-förmigen Grundlinienelements. Die Länge ist bei dem Grundlinienelement die Ausdehnung in einer Richtung senkrecht zur Längsrichtung des Stents und bei dem Stegelement die Ausdehnung entlang des Grundlinienelements.

Im Bereich der jeweiligen Extrema (Minimum, Maximum) in der Krümmung entlang der gedachten Grundlinie 17 sind Verbindungsstege 18 angeordnet, welche jeweils den Steg eines ersten Abschnitts 12 mit dem Steg 14 eines benachbarten Abschnitts 12 verbinden. Die im Wesentlichen in Längsrichtung des Stents verlaufenden Verbindungsstege 18 dienen dazu, die Kräfte, die auf den Stent in Längsrichtung (Richtung der Längsachse) wirken, aufzunehmen. Jeder Verbindungssteg 18 bildet vorzugsweise einen Wellenbogen aus, der jeweils einen Berg und ein Tal aufweist bzw. die Krümmungsradien abwechselnd entgegengesetzt in eine Richtung senkrecht zur Längsrichtung des Stents verlaufen. Es können unterschiedliche Typen von Verbindungsstegen 18 an dem gleichen Stent verwendet werden, z.B. zwei verschiedene Typen von Verbindungsstegen 18 mit unterschiedlichen Krümmungsradien, z.B. ein erster Typ von Verbindungsstegen 18 zwischen dem ersten Abschnitt 12 mit einem Steg 14 und dem zweiten Abschnitt 12 mit einem Steg 14, ein zweiter Typ von Verbindungsstegen 18 zwischen dem zweiten Abschnitt 12 und einem dritten Abschnitt 12 mit einem Steg 14 sowie ein erster Typ von Verbindungsstegen 18 zwischen dem dritten Abschnitt 12 und einem vierten Abschnitt 12 mit einem Steg 14 usw.

Für dieses und alle nachfolgend beschriebenen Ausführungsbeispiele kann der Stent als sogenannter Slotted-Tube-Stent hergestellt werden. Dies bedeutet, dass die Hohlräume zwischen den einzelnen Stegen mittels eines Lasers aus dem Ausgangsmaterial, einem Rohr, herausgetrennt werden. Alternativ kann der Stent als sogenannter Braided-Stent gefertigt werden. Dabei wird der Stent aus Draht geflochten. Es sind jedoch auch andere Herstellungsarten denkbar, z.B. das Verschweißen von Einzelteilen des Stents oder Rapid-Prototyping.

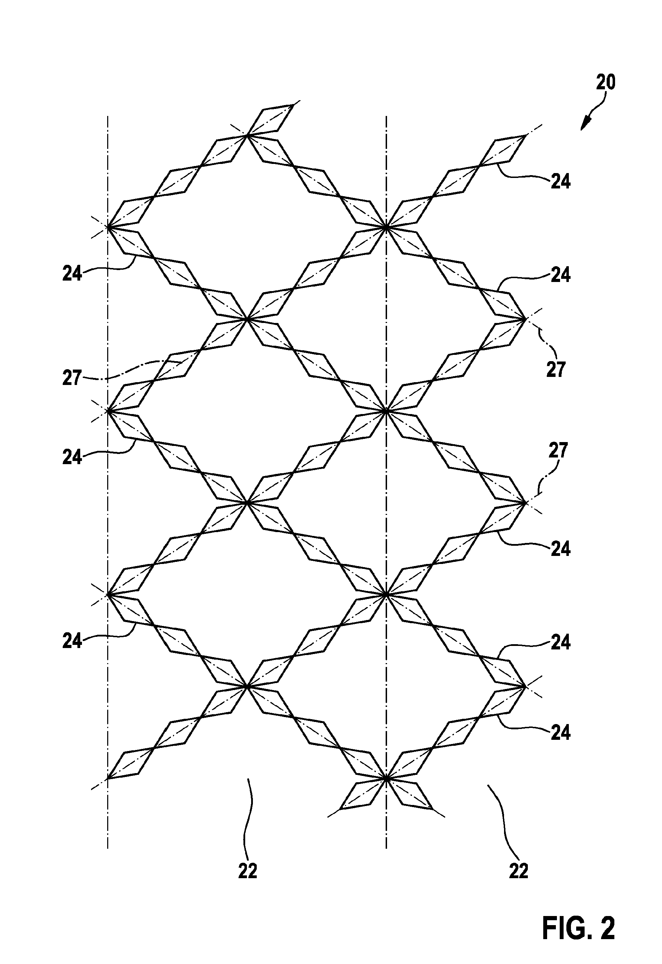

In Fig. 2 ist die Abwicklung eines zweiten Ausführungsbeispiels eines Stents dargestellt. Jeder ringförmiger Abschnitt 22 weist Stege 24 auf, die rhombenförmigen Maschen entlang von gedachten Grundlinien 27 (gestrichelte Linien) bilden. In der Darstellung sind die ringförmigen Abschnitte 22 voneinander durch jeweils eine Strich-Punkt-Linie abgegrenzt.

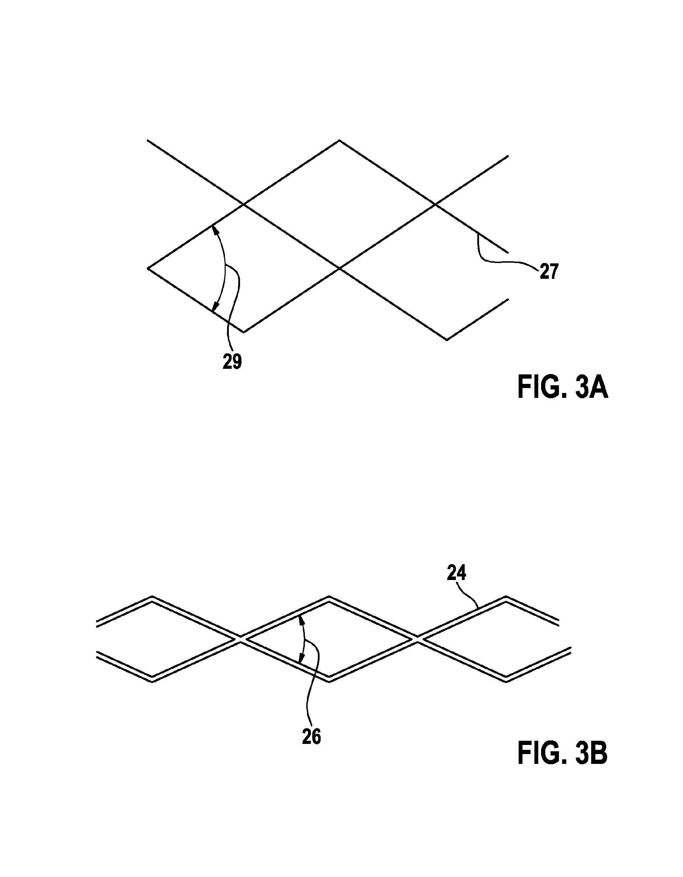

An jeder Seite der Rhomben aus den gedachten Grundlinien 27 verlaufen die Stege 24, welcher aus aneinander gereihten kleinen Rhomben bestehen. Jeder kleine Rhombus bildet ein Stegelement. Die Form der Grundlinienelemente (Rhombenmaschen) und der Elemente der Stege 24 sind noch einmal einzeln in Fig. 3 dargestellt, wobei Fig. 3a) die Anordnung der gedachten Grundlinien erster Ordnung 27 veranschaulicht, während Fig. 3b) die Stege 24 in vergrößerter Darstellung zeigt. Die gedachten Grundlinien 27 bilden eine Mittellinie durch die Rhomben-Elemente der Stege 24.

Mit Hilfe von Fig. 3 wird auch deutlich, dass das zweite Ausführungsbeispiel eine Variante darstellt, bei der die Grundlinienelemente (Rhomben) nicht im strengen mathematisch-geometrischen Sinne ähnlich zu den Rhomben der Stege 24 sind. Dies liegt daran, dass der spitze Innenwinkel 26 der Rhomben der Stege 24 kleiner ist als der spitze Innenwinkel 29 der Rhomben, die die gedachte Grundlinien 27 ausbilden. Das zweite Ausführungsbeispiel wird daher als fraktalähnliche Struktur bezeichnet.

Die Länge der Rhomben der Stege 24 beträgt etwa ein Sechstel der Länge der Rhomben, die die Grundlinie ausbilden. Die Länge ist bei dem Grundlinienelement die Ausdehnung in Längsrichtung des Stents und bei dem Stegelement die Ausdehnung entlang der jeweiligen Seite des Grundlinienelements.

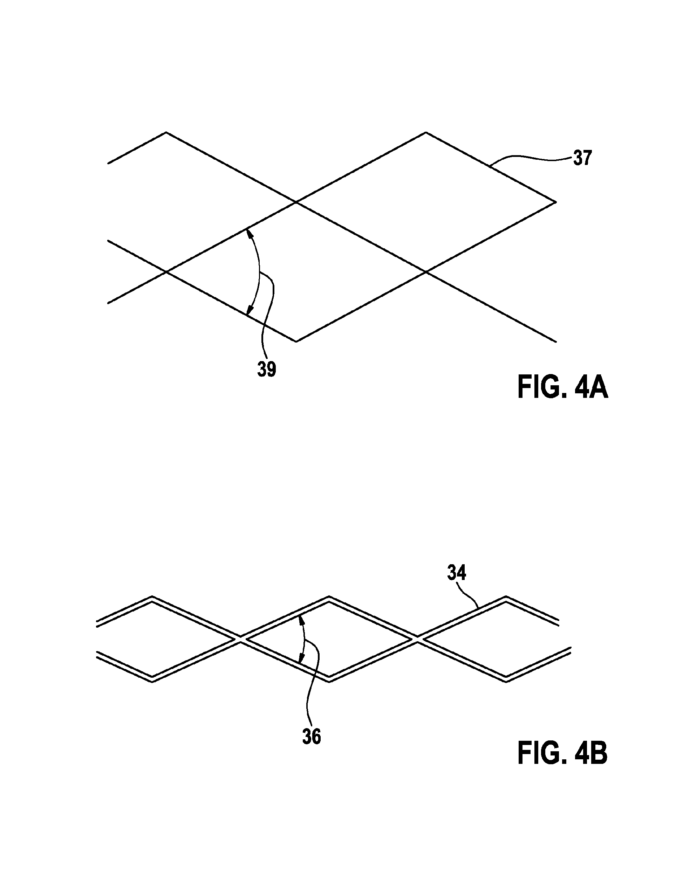

In Fig. 4 ist demgegenüber ein drittes Ausführungsbeispiel dargestellt, bei dem die Rhomben der Stege 34 im mathematischen Sinne ähnlich den Rhomben sind, aus denen sich die gedachten Grundlinien 37 zusammensetzen. Die Ähnlichkeit der Rhomben wird dadurch bestimmt, dass der spitze Innenwinkel 36 der Stegelemente gleich groß ist wie der spitze Innenwinkel 39 der Rhomben der Grundlinie 37. Ein Implantat mit den Stegen 34, welche analog zu Fig. 2 entlang der gedachten Grundlinien 37 angeordnete werden, bildet folglich eine fraktale Struktur aus.

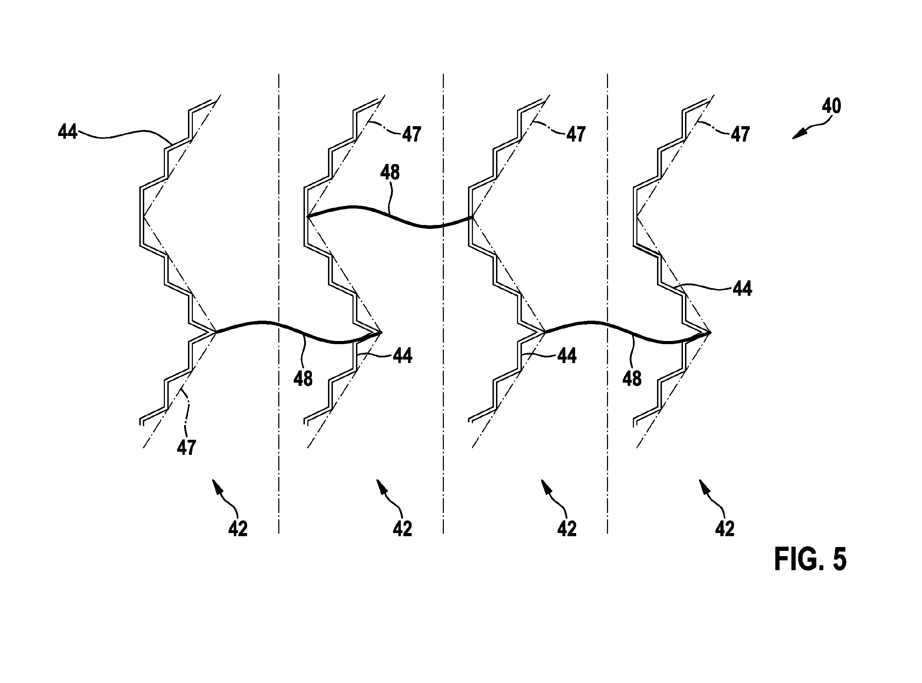

Fig. 5 zeigt ein viertes Ausführungsbeispiel, bei dem in jedem ringförmigen Abschnitt 42 des Stents 40 Stege 44 angeordnet sind, welche sich aus nebeneinander angeordneten Zick-Zack-Elementen zusammensetzen, jedes Element enthält dabei zwei benachbarte Seiten eines Dreiecks. Die Zick-Zack-Elemente des jeweiligen Stegs 44 sind entlang einer gestrichelt gezeichneten, gedachten Grundlinie 47 angeordnet, welche im gesamten Abschnitt 42 auf der rechten Seite der Zick-Zack-Elemente des Stegs 44 verläuft. Jede gedachte Grundlinie 47 besteht ebenfalls aus nebeneinander angeordneten Zick-Zack-Elementen. Analog zum ersten Ausführungsbeispiel sind im Bereich der jeweiligen Minima oder Maxima der Stege 44 S-förmige Verbindungsstege 48 vorgesehen, welche jeweils einen Steg 44 eines ersten Abschnitts 42 mit einem Steg 44 eines benachbarten Abschnitts 42 verbinden und im Wesentlichen in Längsrichtung verlaufen.

Die Länge eines zick-zack-förmigen Stegelements beträgt ungefähr ein Sechstel der Länge eines zick-zack-förmigen Grundlinienelements. Die Länge ist bei dem Grundlinienelement die Ausdehnung in einer Richtung senkrecht zur Längsrichtung des Stents und bei dem Stegelement die Ausdehnung entlang des Grundlinienelements.

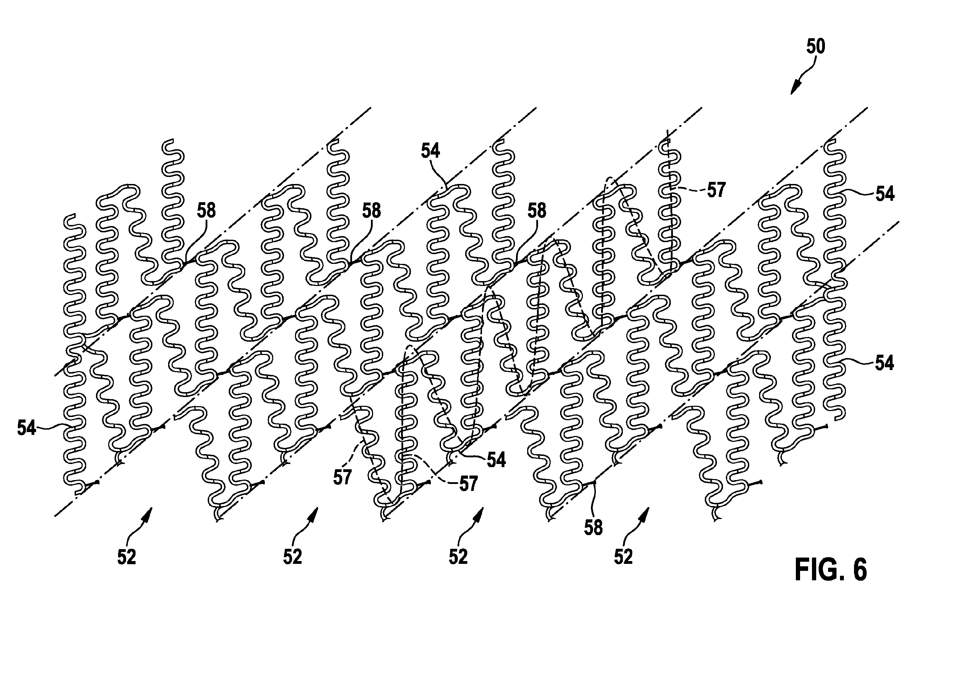

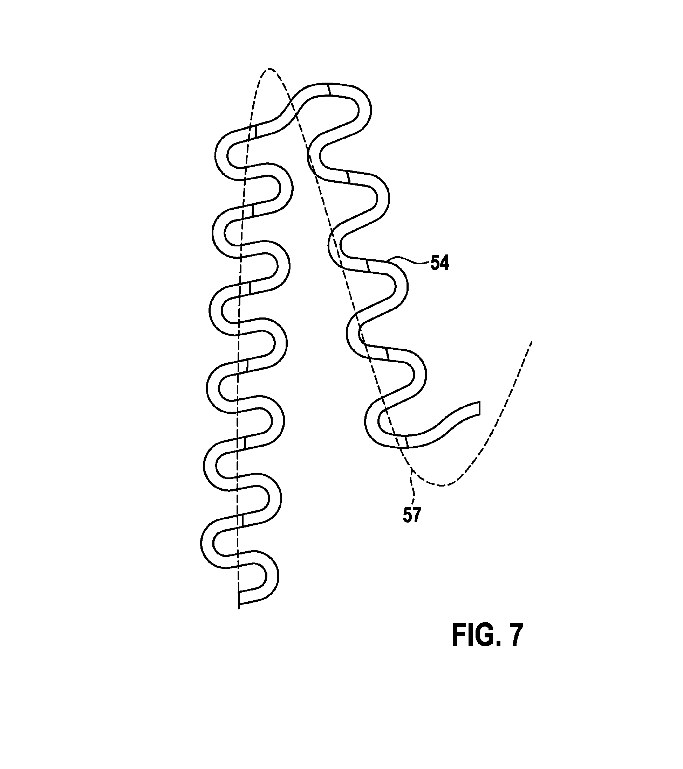

Das in den Figuren 6 und 7 dargestellte fünfte Ausführungsbeispiel weist Stege 54 auf, die entlang einer gedachten Grundlinie 57 nebeneinander angeordnete wellenbogenförmige Elemente vorsieht. Die gedachte (gestrichelt gezeichnete) Grundlinie setzt sich ebenfalls aus wellenbogenförmigen Grundlinienelementen, die nebeneinander angeordnet sind, zusammen. Besonders deutlich zeigt der in Fig. 7 dargestellte Ausschnitt, dass die Elemente des Stegs 54 lediglich im Wesentlich ähnlich zu der Form der Elemente der Grundlinie 57 sind. Die Grundlinie 57 bildet im Wesentlichen eine Mittellinie für die Anordnung der Elemente des Stegs 54. Auch bei dieser Ausführungsform werden die Stege 54 benachbarter helixförmiger Abschnitte 52 über kurze, im Wesentlichen in Längsrichtung verlaufende Verbindungsstege 58 miteinander verbunden.

Die Länge eines wellenbogen-förmigen Stegelements des in dem anhand der Fig. 6 und 7 dargestellten Ausführungsbeispiels beträgt ungefähr ein Zwölftel der Länge eines wellenbogen-förmigen Grundlinienelements. Die Länge ist bei dem Grundlinienelement die Ausdehnung in einer Richtung schräg zur Längsrichtung des Stents (siehe strichpunktierte Linien) und bei dem Stegelement die Ausdehnung etwa entlang des Grundlinienelements.

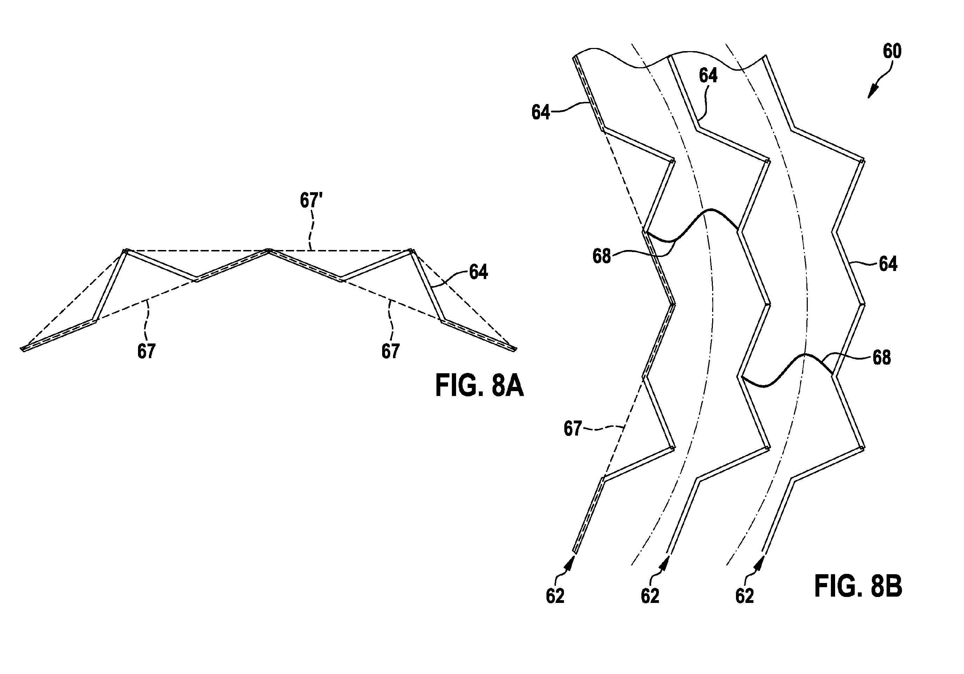

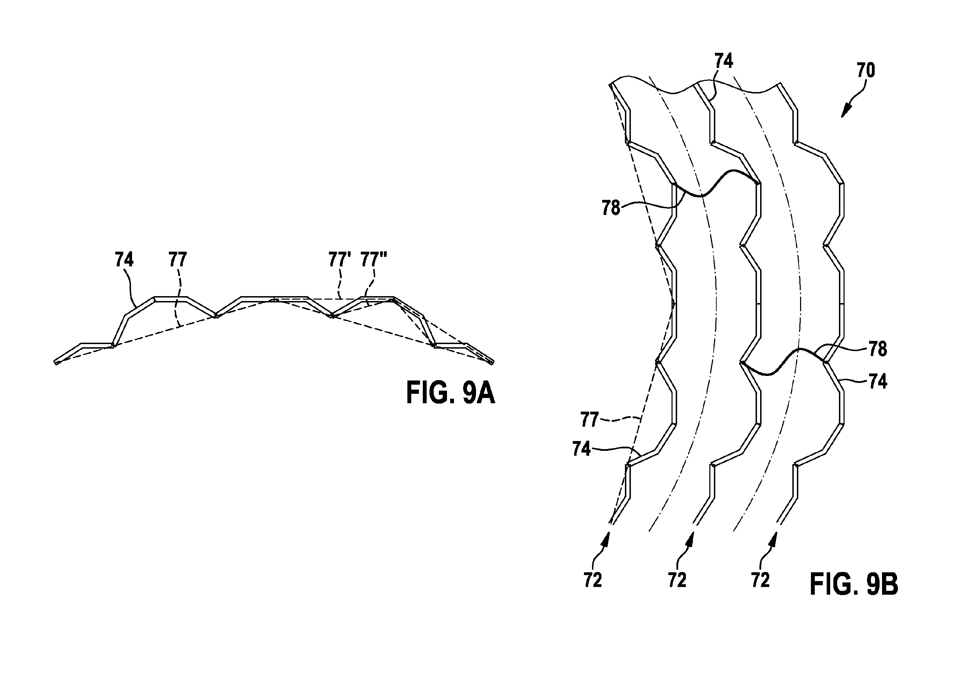

Die in den Figuren 8 und 9 gezeigten sechsten und siebenten Ausführungsbeispiele enthalten weitere Fraktalebenen.

Bei dem in Fig. 8 gezeigten sechsten Ausführungsbeispiel wurde ausgehend von einer aus aneinander gereihten Zick-Zack-Elementen gebildeten Grundlinie erster Ordnung 67 eine gedachte Grundlinie zweiter Ordnung 67' gebildet, welche sich ebenfalls aus den Zick-Zack-Elementen zusammensetzt, die jedoch nur 50% der Größe der Zick-Zack-Elemente der Grundlinie erster Ordnung 67 aufweisen. Entlang dieser gedachten Grundlinie zweiter Ordnung 67' werden nun die ebenfalls Zick-Zack-förmigen Elemente des Stegs 64 angeordnet. Diese weisen gegenüber den Zick-Zack-Elementen der Grundlinie erster Ordnung 67 nur eine Größe von etwa 25% auf. Die Zick-Zack-Elemente der Grundlinie erster Ordnung 67 sind ähnlich zu den Zick-Zack-Elementen der gedachten Grundlinie zweiter Ordnung 67' und zu den Zick-Zack-Elementen des Stegs 64.

Die Länge eines zick-zack-förmigen Stegelements beträgt ungefähr ein Viertel der Länge eines zick-zack-förmigen Elements der Grundlinie erster Ordnung 67. Die Länge ist bei dem Grundlinienelement der Grundlinie erster Ordnung 67 die Ausdehnung in einer Richtung senkrecht zur Längsrichtung des Stents und bei dem Stegelement die Ausdehnung entlang des Grundlinienelements der Grundlinie zweiter Ordnung 67'. Die Länge eines zick-zack-förmigen Elements der Grundlinie zweiter Ordnung 67' beträgt ungefähr die Hälfte der Länge eines zick-zack-förmigen Elements der Grundlinie erster Ordnung 67. Die Länge ist bei dem Element der Grundlinie zweiter Ordnung 67' die Ausdehnung entlang des jeweiligen Elements der Grundlinie erster Ordnung 67.

Bei dem in Fig. 9 dargestellten Ausführungsbeispiel eines erfindungsgemäßen Implantats wird noch eine weitere Fraktalebene hinzugefügt. Aus einer gedachten Grundlinie erster Ordnung 77 wird eine gedachte Grundlinie zweiter Ordnung 77' analog zu dem Vorgehen des in Fig. 8 gezeigten Ausführungsbeispiels gebildet. Analog dazu wird aus der Grundlinie zweiter Ordnung 77' eine gedachte Grundlinie dritter Ordnung 77" gebildet, wobei die Elemente der Grundlinie dritter Ordnung 77" ähnlich sind zu den Zick-Zack-Elementen der Grundlinie erster Ordnung 77 und den Elementen des Stegs 74. Die gedachte Grundlinie dritter Ordnung 77" dient als Basislinie für die Anordnung (nebeneinander) der Zick-Zack-förmigen Elemente des Stegs 74. Die Zick-Zack-Elemente der Stege 74 weisen eine Größe von etwa 12,5% verglichen mit der Größe der Elemente der gedachten Grundlinie erster Ordnung 77 auf. Jeder Steg 74 ist in einem ringförmigen Abschnitt 72 des Implantats 70 angeordnet.

Die Länge eines zick-zack-förmigen Elements des Stegs 74 beträgt ungefähr ein Achtel der Länge eines zick-zack-förmigen Elements der Grundlinie erster Ordnung 77. Die Länge ist bei dem Grundlinienelement der Grundlinie erster Ordnung 77 die Ausdehnung in einer Richtung senkrecht zur Längsrichtung des Stents und bei dem Stegelement die Ausdehnung entlang des Grundlinienelements der Grundlinie dritter Ordnung 77". Die Länge eines zick-zack-förmigen Elements der Grundlinie zweiter Ordnung 77' beträgt ungefähr die Hälfte der Länge eines zick-zack-förmigen Elements der Grundlinie erster Ordnung 77 und die Länge eines zick-zack-förmigen Elements der Grundlinie dritter Ordnung 77" beträgt ungefähr ein Viertel der Länge eines zick-zack-förmigen Elements der Grundlinie erster Ordnung 77. Die Länge ist bei dem Element der Grundlinie zweiter Ordnung 77' die Ausdehnung entlang des jeweiligen Elements der Grundlinie erster Ordnung 77 und bei dem Element der Grundlinie dritter Ordnung 77" die Ausdehnung entlang des jeweiligen Elements der Grundlinie zweiter Ordnung 77'.

Die Stege 74 benachbarter Abschnitte 72 werden mittels S-förmigen Verbindungsstegen 78 in Längsrichtung miteinander verbunden. Analog dazu weist auch das in Fig. 8 gezeigte sechste Ausführungsbeispiel Verbindungsstege 68 auf, welche die Stege 64 benachbarter Abschnitte 62 in Längsrichtung miteinander verbinden.

### Bezugszeichenliste

- 10, 20, 30, 40, 50, 60, 70: Stent
- 12, 22, 32, 42, 52, 62, 72: Abschnitt
- 14, 24, 34, 44, 54, 64, 74: Steg
- 17, 27, 37, 47, 57, 67, 77: gedachte Grundlinie erster Ordnung
- 67', 77': gedachte Grundlinie zweiter Ordnung
- 77": gedachte Grundlinie dritter Ordnung
- 18,48,58,68,78: Verbindungssteg

## Patentansprüche

1. Implantat mit einer durchbrochenen, vorzugsweise hohlzylinderförmigen Grundstruktur, welche mindestens einen Abschnitt aufweist, wobei in jedem Abschnitt mindestens ein Steg angeordnet ist, **dadurch gekennzeichnet, dass** der Steg eine Vielzahl von gleichen, einfachen und miteinander verbundenen Elementen aufweist, deren Grundform zu der Form von Grundlinienelementen mindestens einer gedachten Grundlinie erster Ordnung, die ebenfalls in dem Abschnitt verläuft, im Wesentlichen ähnlich ist, wobei die mindestens eine gedachte Grundlinie erster Ordnung eine Bezugslinie für die Anordnung der Elemente darstellt und die Elemente höchstens halb so groß sind wie die Grundlinienelemente.

2. Implantat nach Anspruch 1, **dadurch gekennzeichnet, dass** der mindestens eine Abschnitt ringförmig oder helixförmig ist.

3. Implantat nach einem der vorhergehenden Ansprüche, **dadurch gekennzeichnet, dass** der mindestens eine Steg eines Abschnitts oder eines Bereichs des Abschnitts mit dem mindestens einen Steg des benachbarten Abschnitts oder eines benachbarten Bereichs des Abschnitts mittels mindestens eines Verbindungsstegs, der vorzugsweise S-förmig ausgebildet ist, verbunden ist.

4. Implantat nach einem der vorhergehenden Ansprüche, **dadurch gekennzeichnet, dass** ein Element und ein Grundlinienelement die Form eines Wellenform-Bogens, eines Rhombus, eines Dreiecks, eines Zick-Zack-Elements oder dergl. aufweist.

5. Implantat nach einem der vorhergehenden Ansprüche, **dadurch gekennzeichnet, dass** die Elemente entlang der mindestens einen gedachten Grundlinie erster Ordnung angeordnet sind.

6. Implantat nach einem der vorhergehenden Ansprüche, **dadurch gekennzeichnet, dass** mindestens eine gedachte Grundlinie zweiter Ordnung vorgesehen ist, welche die Grundlinienelemente aufweist, welche mit einer Größe von höchstens 50% entlang der Grundlinie erster Ordnung angeordnet sind, und die Elemente entlang der mindestens einen gedachten Grundlinie zweiter Ordnung angeordnet sind.

7. Implantat nach einem der vorhergehenden Ansprüche, **dadurch gekennzeichnet, dass** mindestens eine gedachte Grundlinie zweiter Ordnung vorgesehen ist, welche die Grundlinienelemente aufweist, die mit einer Größe von höchstens 50% entlang der Grundlinie erster Ordnung angeordnet sind, und dass ferner mindestens eine Grundlinie dritter Ordnung vorgesehen ist, welche Grundlinienelemente aufweist, die mit einer Größe von höchstens 25% entlang der Grundlinie zweiter Ordnung angeordnet sind, und die Elemente entlang der mindestens einen gedachten Grundlinie dritter Ordnung angeordnet sind.

8. Implantat nach einem der Ansprüche 5 bis 7, **dadurch gekennzeichnet, dass** die Elemente oder Grundlinienelemente derart entlang der mindestens einen Grundlinie erster Ordnung oder der mindestens einen Grundlinie zweiter Ordnung oder der mindestens einen Grundlinie dritter Ordnung angeordnet sind, dass die jeweilige Grundlinie eine Zentrallinie oder eine obere Begrenzungslinie oder eine untere Begrenzungslinie darstellt.
